# EUROPEAN PATENT SPECIFICATION

(11) **EP 0 578 102 B1**
(45) Date of publication and mention of the grant of the patent: **18.02.1998**
(21) Application number: 93110340.2
(22) Date of filing: 29.06.1993
(51) Int. Cl.: A61B 17/00, A61B 19/02

(54) **Dispenser for accessories for endoscopic surgical instrument**
Spender für Zubehör für endoskopisches chirurgisches Instrument
Distributeur d'accessoires pour instrument chirurgical endoscopique

(30) Priority: 30.06.1992 US 906673
(43) Date of publication of application: 12.01.1994
(73) Proprietor: United States Surgical Corporation, Norwalk, Connecticut 06856 (US)
(72) Inventor: Matula, Paul A., Brookfield, CT 06084 (US); McCabe, William J., New Canaan, CT 06840 (US); McCourtney, Gerald, Orono, Minnesota 55391 (US); Gemmell, Dennis, Robinsdale, Minnesota 55426 (US); Hamlin, Jerry, Golden Valley, Minnesota 55427 (US)
(74) Representative: Marsh, Roy David

(56) References cited:
- WO-A-90/03152
- WO-A-92/00705
- US-A- 4 120 397
- US-A- 4 657 016

## Description

### BACKGROUND OF THE INVENTION

### Field of the Invention

The present invention relates to endoscopic surgical instruments, and more particularly to endoscopic surgical instruments having a plurality of interchangeable tool tip members for performing surgical procedures. The invention has particular application to instruments for aspirating and irrigating a surgical site.

### 2. Discussion of the Prior Art

Surgical devices for providing irrigation fluid and suction to a surgical site to irrigate and evacuate the tissue in the area on which the surgical procedure is being performed are well known in the art. Several of these devices provide a handle member having switching means for turning on and off the flow of the fluid stream and the suction means, and typically connect the suction source and the fluid source to an elongated flexible tubular member which is positioned adjacent the surgical site. In many instances, the tube or catheter is comprised of a complex series of passages which provide a separate channel for the irrigation fluid and a separate channel for the suction means. Several devices provide a pump source to provide the fluid under pressure; however, other devices provide a source of irrigation fluid which is operable under head pressure to gently wash the tissue. The prior art devices typically provide a large tube or catheter which enclose the several channels to deliver the fluid and provide the suction during oral surgery, or invasive surgery which allows for the positioning of the cumbersome tubing.

Several of the prior art devices provide numerous features including electrocautery, laser dissection, and viewing capabilities, all through a single tool member disposed at the operative distal end of the device. Typically, the handle grip includes on/off switches in the form of trumpet valves which allow the surgeon to selectively choose the suction or irrigation feature. Many devices provide a pistol-type hand grip which allows the surgeon to operate the device with the thumb-actuated valves. Other devices provide tubular connections such as Luer-type connectors to couple the irrigation source or the suction source to the catheter or tube.

With the recent developments in endoscopic and laparoscopic surgical procedures, it is necessary to provide a device in which many of the functions provided by the more complex and cumbersome prior art devices are included in a streamlined construction in which many of the features are provided in a single unit. In laparoscopic and endoscopic surgical procedures, a small incision or puncture is made in the patient's body to provide access for a tube or cannula device. The cannula is inserted into the patient's body through the provision of a trocar assembly which further includes an obturator for penetrating the body wall. After the obturator is removed, the cannula remains in place to maintain access to the surgical site. Once the cannula is in place, the surgical instrument may be inserted through the cannula to perform the procedure, while the surgical area is viewed through an endoscope or a miniature camera inserted through secondary cannulas to display the procedure on a video monitor.

The prior art devices are subject to several disadvantages when considered for use in laparoscopic or endoscopic surgical procedures. The primary focus behind such surgical procedures is that the surgery is minimally invasive to the patient's body, consequently reducing damage to surrounding tissue and organs and reducing the scarring resulting from the operation, which, as a result, greatly reduces recovery time for the patient. The prior art devices, which typically provide a plurality of channels in the tube or catheter portion to transport the suction and irrigation means to the surgical site, are generally provided for invasive type surgery which allows the larger diameter catheters to be manually positioned adjacent the surgical objective through large incisions.

A further limitation to which the prior art devices are subjected involves positioning of the device during the surgical procedure. Many of these devices are provided with a pistol-type grip which requires a particular orientation of the device in relation to the surgeon's position during the procedure. Should it become necessary for the device to be relocated during the surgical procedure, it is often times uncomfortable to the surgeon to position the device at an angle that does not facilitate operation of the valve members to turn the various features on and off. As a result, the effectiveness of the device is limited, and in many times requires a surgical assistant to operate the device for the surgeon.

Typical suction and irrigation devices having a hand grip in the shape of a pistol are disclosed in U.S. Patent No. 4,149,315 to Page, Jr. et al. and U.S. Patent No. 4,776,840 to Freitas et al. Page, Jr. et al. provides a dental suction/irrigation device which includes an elongated tube member which transports the suction means and the irrigation means to the tissue site. The elongated tubular member comprises a pair of concentric tubes where the inner tube provides the irrigation fluid and the outer tube is provided for the suction. A pair of trumpet valves are provided to actuate the irrigation source and the aspiration source. Freitas et al. discloses a similar device but includes a complex internal manual pump to provide the irrigation fluid. A second flexible tube is provided for a vacuum source to evacuate fluid and gases from the surgical site.

U.S. Patent No. 4,744,360 Bath provides a surgical device for removing cataract lenses which includes an optical fiber for laser surgery which is surrounded by an irrigation sleeve and a separate aspirator sleeve which provides fluid for irrigation and suction for evacuation, respectively, of the surgical site.

A Cabot Medical Corporation brochure (copyright 1990) discloses a suction/irrigation probe which includes a hydrodissection insert which comprises a rod which passes through the tube of the suction/irrigation probe to adjust the flow of the irrigation fluid.

Other known devices include U.S. Patent No. 4,921,476 and U.S. Patent No. 4,493,694 to Wuchinich, and U.S. Patent No. 3,527,203 to Gravlee, which include a tube having several channels for carrying the irrigation fluid separately from the suction device.

A further disadvantage to the devices known in the prior art lies in the fact that the operative end of the device includes only one, non-changeable tool member, if a tool member is provided at all. Typically, suction and irrigation devices terminate in an open tube member for aspirating or irrigating the surgical site. Those devices which do provide a tool at the operative end generally have a permanent member in position, so that the surgeon must change instruments in the event a different tip is required. In the field of dentistry, WO 92/00705 discloses a receptacle for drill bit or dental pins. The receptacle can be circular with the bits or pins arranged around the circumference and a rotatable lid to expose one bit or pin at a time for removal from the receptacle. No means for uncoupling the dental pins from the drill bit are provided to the receptacle. Other prior art devices have interchangeable tool members, however each tool is attached to a different tubular member so that the entire tubular member needs to be replaced. This causes difficulty in changing tools due to the maneuvering and hand manipulation required to switch tubular members, and further increases the expense of the instruments.

The invention aims to provide a novel endoscopic surgical device for suction and irrigation of tissue during a surgical procedure which obviates the disadvantages encountered in the prior art and provides a compact instrument which includes many of the features necessary to perform the surgical procedure, and which is dimensioned to fit through a cannula for the performance of endoscopic or laparoscopic surgical procedures. In preferred embodiments, the device of the present invention allows a surgeon to operate the suction and irrigation device with either hand and at any orientation to the surgical site comfortably and without assistance, and further allows the surgeon to expand the surgical procedure through the provision of a plurality of interchangeable tool tip members for quick connection to the surgical device. The tool tips preferably are provided in an indexable case to allow the surgeon to choose the right tool tip for the required surgical procedure.

### SUMMARY OF THE INVENTION

The invention is set out in claim 1 below.

The present invention aims to provide a novel irrigation and aspiration device for performing endoscopic or laparoscopic surgical procedures which allows the surgeon to operate the device with either hand and at any orientation to the patient's body. The device may include numerous features necessary for the performance of a surgical procedure such as dissection of tissue, or to provide suction and irrigation to a surgical site where the procedure is performed with additional instruments. The present invention may further provide a plurality of interchangeable tip members which may be freely substituted at the operative end of the device by the surgeon depending on the procedure being performed. The tips preferably are packaged in an indexable case, which provides sterile and efficient access to the tips by the surgeon.

The suction and irrigation device of the present invention comprises a variably orientable subassembly which may be incorporated into various outer enclosures or housings dependent on the surgeon's preference and on the type of surgical procedure in which the surgical instrument is to be used. The subassembly essentially comprises a connection port for a source of suction and means to actuate the source of suction through the port, as well as a connection point for irrigation fluid with means to actuate the irrigation source through the port. A single lumen cannula is provided which communicates with the actuating means for both the suction port and the irrigation port which transports the suction means and the irrigation fluid to the surgical site. The device may further include a port for an optical fiber for the performance of laser surgery which further communicates with the single lumen cannula to locate the optical fiber through the cannula to the surgical site.
Furthermore, electrocautery means may be provided for the performance of cauterization procedures at the surgical site.

The single lumen cannula is provided with a connection means at its distal end for the interchangeable connection of various operative tips which allows the surgeon to perform various surgical procedures. These tips include, among others, a surgical knife, blunt dissectors, retractors, spatulas, and a nozzle for high pressure hydrodissection. The cannula is provided with a plurality of apertures at the distal end for communicating the interior of the cannula with the surrounding environment at the surgical site.

It is often necessary during a surgical procedure for a surgeon to change the tool mechanism at the surgical site. In many instances, the change of tools must be done quickly in order to minimize trauma at the site. In the past, it was often necessary to provide a multitude of surgical instruments or tubular members with tools attached in order to be properly prepared for whatever may arise during the operation. A need therefore exists for the provision of a single instrument having interchangeable tip members at the operative end to provide a convenient and quick to use multi-surgical instrument.

To this end, the present invention includes a dispenser for the tool tip members to allow the surgeon to choose the proper tip for the required surgical operation. The dispenser may hold a plurality of tips and includes means for dispensing the tips to the surgeon as well as means to retain the remaining tips. The means for dispensing includes means for inserting the surgical device into the dispenser to snap on the tip member, so that sterility is maintained and the surgeon may quickly and efficiently access the tips. It is also contemplated that the dispenser and suction/irrigation device be packaged together as a kit, to enable the surgeon to have the proper tip members on hand during the operation.

A further optional feature of the device is the hydrodissection capability, in which the high pressure fluid may be directed to the tissue at high pressure to dissect the tissue. In this regard, a concentric outer sleeve member is provided along the length of the cannula which is longitudinally slidable to cover one or all of the apertures at the distal end of the cannula. This allows the surgeon to vary the pressure and provides a visual indication for adjusting the pressure at the distal end.

A further optional feature of the present invention is the variably orientable valve members which allows the surgeon to operate the device with either hand and at any orientation in relation to the patient's body. Preferably, the optical fiber connection port is axially aligned with the longitudinal axis of the single lumen cannula. At least one of the connection ports for either the aspiration means or the suction means, or both, includes a rotatable trumpet valve to allow the surgeon to rotate the valve at least 180° from one position perpendicular to the longitudinal axis of the single lumen cannula to a second position perpendicular to the longitudinal axis of the single lumen cannula on the opposite side of the cannula. If one connection port is rotatable, the other may be rotatable, or may extend from the distal end of the device so that the connection port is within substantial parallel alignment with the longitudinal axis of the single lumen cannula and the laser optical fiber connection port. Furthermore, the connection for the electrocautery feature preferably includes a *b*ayonet-type male connector which extends at an angle to, but generally in the same direction as, the longitudinal axis of the single lumen cannula. These features allow the surgeon to vary the orientation of the device and operate the device with either hand by rotating the trumpet valves so that the tubes or hoses which deliver the suction means or the irrigation fluid are in an unobstructed position.

### BRIEF DESCRIPTION OF THE DRAWINGS

The foregoing features of the present invention will become more readily apparent and may be understood by referring to the following detailed description of one embodiment of endoscopic surgical aspiration and irrigation instrument, taken in conjunction with the accompanying drawings, in which:
Figure 1 illustrates a perspective view of the subassembly of the endoscopic surgical instrument for aspiration and irrigation which may form part of the present invention;
Figure 2 illustrates a perspective view including an external housing according to a first embodiment;
Figure 3 illustrates a side plan view in partial cross section of the embodiment of Figure 2;
Figure 4 illustrates a rear plan view of the device of Figure 2;
Figure 5 illustrates a perspective view of a second embodiment of a subassembly of the endoscopic surgical instrument for aspiration and irrigation which may form part of the present invention;
Figure 6 illustrates a top plan view of the device of Figure 5;
Figure 7 illustrates an exploded perspective view of the valve mechanism of the device of Figure 5;
Figure 8 illustrates a perspective view of a second embodiment of the outer enclosure employing the device of Figure 1 according to the present invention;
Figure 9 illustrates a side plan view in partial cross section of the device of Figure 8;
Figure 10 illustrates a rear plan view of the device of Figure 8;
Figure 11 illustrates a perspective view of the endoscopic surgical instrument for aspiration and irrigation in use during a surgical procedure;
Figures 12a and 12b illustrate an exploded perspective view and a perspective view, respectively, of the distal end of the single lumen cannula having a dissector tool mechanism attached thereto by a bayonet-type clip;
Figures 13a through 13c illustrate various electrocautery dissectors for use with the device of the present invention;
Figures 14a through 14c illustrate various blunt dissectors and surgical knives for use with the device of the present invention;
Figures 15a and 15b illustrate a coupling member for use with the dissectors of Figures 13a through 13c;
Figures 16a and 16b illustrate a coupling member for use with the dissectors of Figures 14a through 14c;

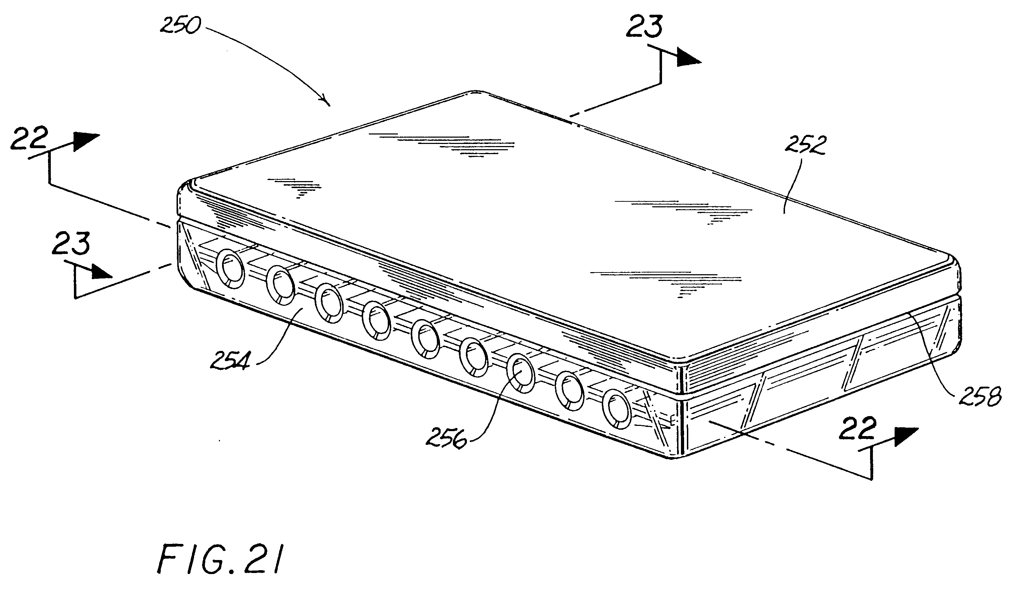

Figures 17 to 20 are cancelled;
Figure 21 illustrates a perspective view of an embodiment of a tip dispenser according to the present invention;
Figure 22 illustrates a cross-sectional view taken along lines 22-22 of Figure 21 of the dispenser of Figure 21;
Figure 23 illustrates a cross-sectional view taken along lines 23-23 of Figure 21 of the dispenser of Figure 21;
Figure 24 is cancelled;
Figures 25a and 25b illustrate an exploded perspective view and a perspective view, respectively, of the distal end of the single lumen cannula having a tool mechanism attached thereto by screw threads;
Figures 26a and 26b illustrate an exploded perspective view and a perspective view, respectively, of the distal end of the single lumen cannula having a tool mechanism attached thereto by a spring clip; and
Figure 27 illustrates a perspective view of various tool mechanisms which can be stored in the dispenser of the present invention.

### DETAILED DESCRIPTION OF THE PREFERRED EMBODIMENTS

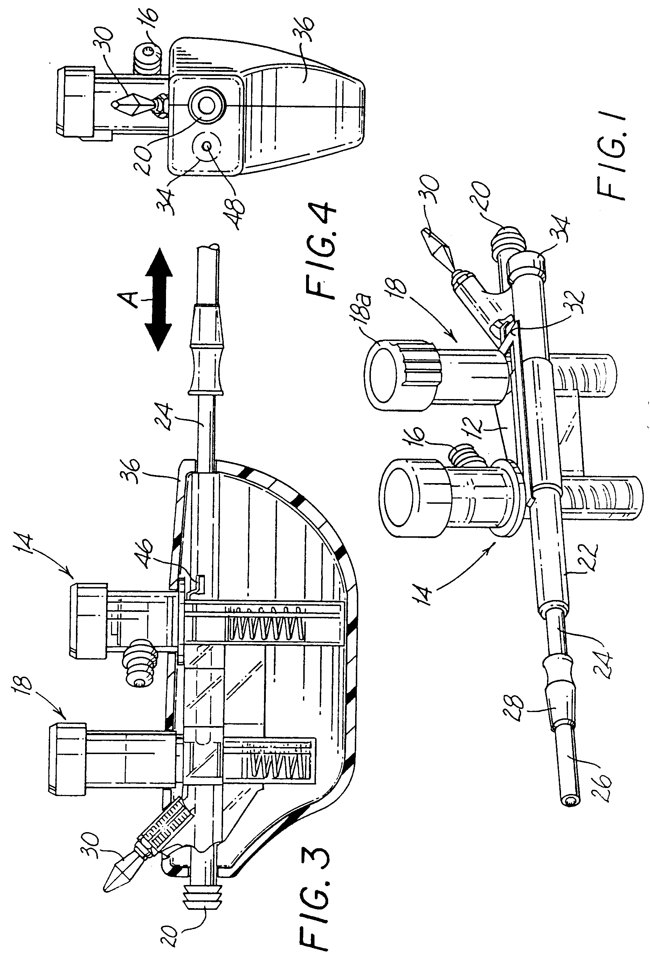

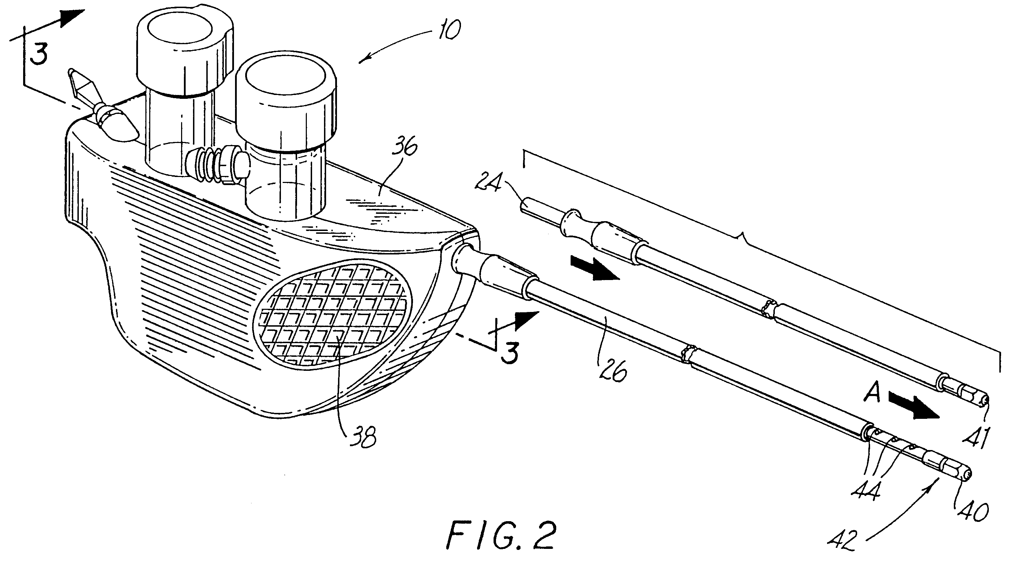

Referring now in specific detail to the drawings, in which like reference numerals identify similar or identical elements throughout the several views, Figure 1 shows an endoscopic surgical instrument for aspiration and irrigation which may form part of the present invention. Instrument 10 comprises a body portion 12 to which at least a pair of valve members 14 and 18 are attached. Preferably, at least one valve member, namely valve member 14, includes a rotatable connection port 16 for coupling a source of irrigation or a source of suction thereto. The function of rotatable connection port 16 will be discussed below. Valve member 18 may include a rotatable connection port; however, in a preferred embodiment valve member 18 includes a rotatably lockable actuator 18a for maintaining a source of constant irrigation or suction. Connection port 20 is controlled by valve member 18.

Body portion 12 essentially comprises a mixing chamber which communicates with both connection port 16 and connection port 20 through valve member 14 and 18, respectively. Body portion 12 extends into coupling member 22 which couples the mixing chamber within body portion 12 with a single lumen cannula 24. Single lumen cannula 24 provides a means for transporting the irrigation fluid or the suction force from their respective sources to the surgical site. Single lumen cannula 24 simplifies the construction of device 10 and significantly reduces cost in that a single tubular member having a reduced diameter single channel therethrough is utilized to carry both the suction and irrigation fluid to the surgical site.

Preferably, single lumen cannula 24 is enclosed within outer sleeve member 26 which concentrically surrounds and contacts single lumen cannula 24 along its length and is slidable in a longitudinal direction by grip member 28. The purpose of the slidable outer sleeve will be discussed below.

Device 10 may further include a bayonet-type connector 30 for providing electrocautery capabilities to device 10. Bayonet connector 30 is in electrical contact with single lumen cannula 24 through the provision of bus bar 32. Bayonet connector 30 provides for cauterization at the surgical site and for electrodissection of tissue. Device 10 may further include laser dissection means, which may be provided by an optical fiber through optical fiber port 34.

Figure 2 illustrates the device of Figure 1 enclosed in a working housing 36 which provides for gripping and handling of device 10. Housing 36 may be provided with scored portion 38 in one or several locations to facilitate gripping. As is seen in Figure 2, single lumen cannula 24 is enclosed by outer sleeve member 26 which is slidable between a proximal position whereby apertures 44 are exposed at the distal end 42, to a distal position where outer sleeve 26 covers apertures 44. A hydrodissection tip 40 is shown as connected to the distal end 42 of single lumen cannula 24. In use, device 10 may be utilized for hydrodissection purposes. In such a case, a high pressure irrigation fluid source is utilized and connected, preferably to either of connection port 16 or connection port 20. As the irrigation fluid exits the aperture 41 at the end of hydrodissection tip 40, the pressure at which the fluid exits may be regulated and varied by sliding outer sleeve 26 in the direction of arrow A to cover one or more of apertures 44. Covering apertures 44 will increase the pressure of the fluid exiting tip 40 to provide for greater or less pressure of the irrigation and dissection fluid.

Preferably, outer sleeve 26 is constructed of an electrical insulating material, such as plastic, or may be provided with an electrically insulating shrink tubing, so that when device 10 is used for electrocautery purposes, the risk of shock is mitigated. Figure 3 shows the electrical connection of bayonet connector 30 with single lumen cannula 24 at connection point 46.

Figure 4 illustrates a rear view of the device of Figure 2 which illustrates connection port 20 as being in axial alignment with valve members 14 and 18, while optical fiber port 34 is in direct axial alignment with single lumen cannula 24. Optical fiber port 34 is provided with a sealing means 48 which generally comprises a rubber type gasket which is penetrable by the optical fiber and seals around the fiber to prevent loss of suction pressure and leakage of irrigation fluid.

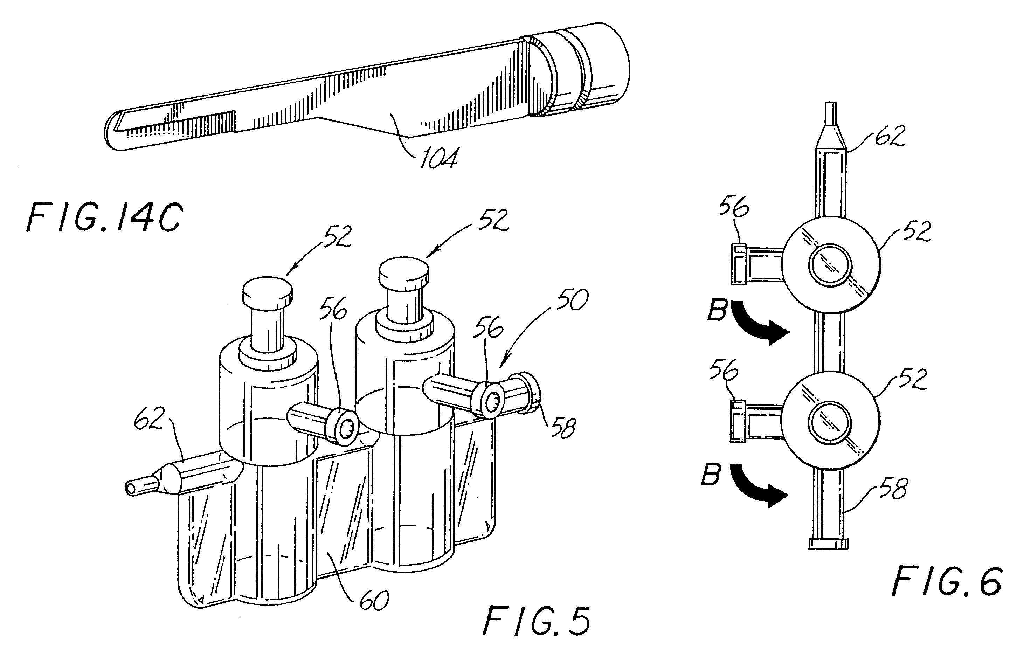

Figures 5 and 6 illustrate an alternate embodiment 50 of the instrument of Figure 1. Instrument 50 comprises a pair of rotatable trumpet valve members 52 which are secured to a body portion 60 and are positioned directly in line with a coupling member 62 which extends into the single lumen cannula described above. An optical fiber port 58 is provided which is directly in line with coupling member 62. Rotatable trumpet valve members 52 include rotatable connection ports 56 whose function will be discussed below.

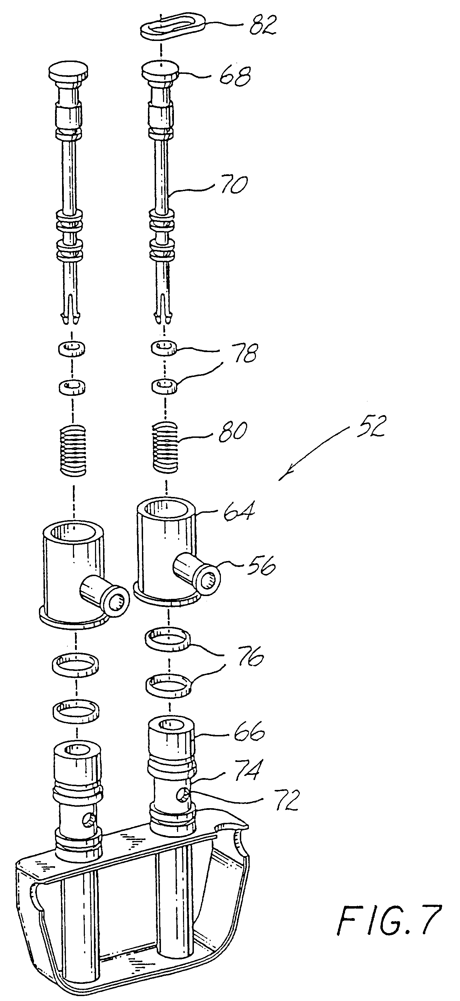

Turning to Figure 7, there is shown the rotatable trumpet valve members 52 (as well as valve member 14 discussed above in connection with Figure 1). Valve members 52 essentially comprise a rotatable outer housing 64 to which connection port 56 is coupled. Outer housing 64 fits over inner housing 66, and valve stem 70 of actuator knob 68 extends through the inner and outer housings. Inner housing 66, is provided with an opening 72 which communicates a chamber as defined by the inner wall of outer housing 64 and chamber wall 74 to allow for the passage of fluid or suction pressure upon actuation of valve member 52. A pair of gaskets 76 are provided which seal the top and bottom of the chamber between the outer housing 64 and the inner housing 66, to prevent leakage while maintaining the rotatable feature. A second pair of gaskets 78 are secured to stem 70 for actuation of valve member 52. A spring means 80 is provided, as is common in trumpet-type valves. In addition, a locking ring 82 may be provided to maintain the valve in the continuously on position. The locking ring may be eliminated such as shown in Figure 1, where a camming surface is provided on the interior surface of actuator knob 18a which engages a cam surface on the outside of outer housing 64.

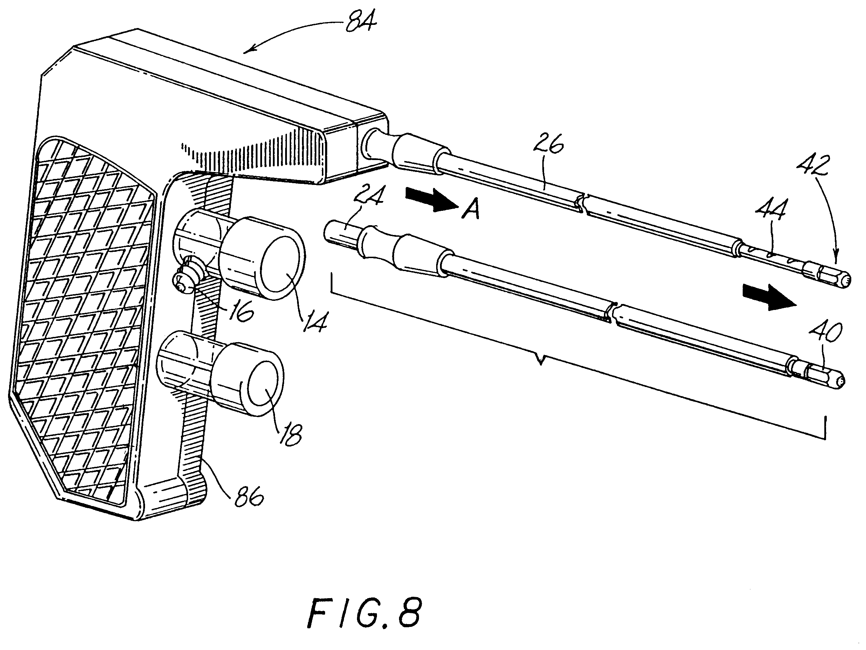

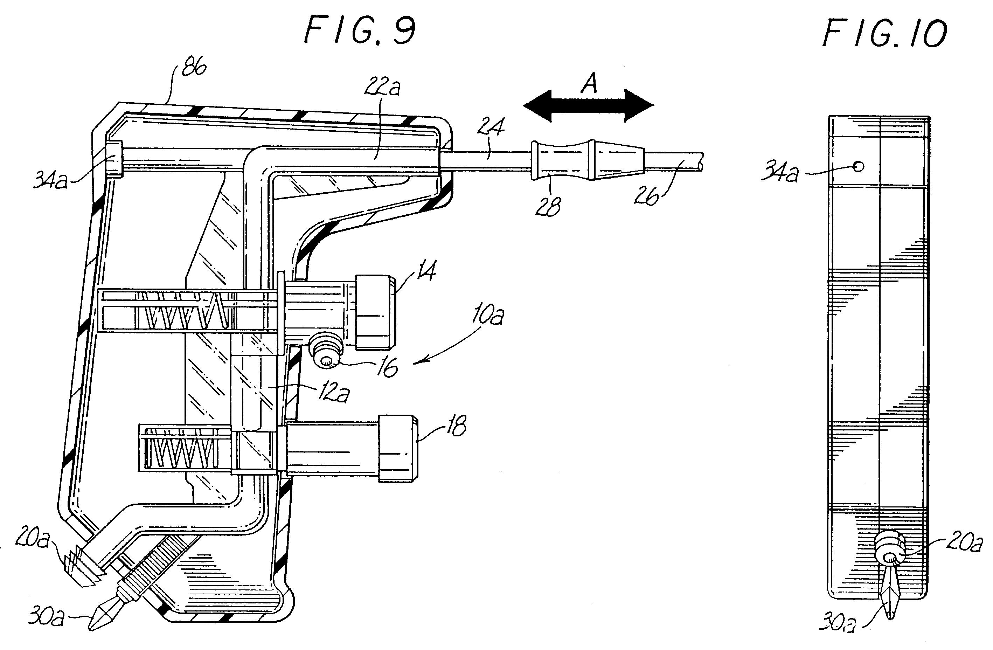

Turning now to Figure 8, there is illustrated a further embodiment of the surgical instrument for aspiration and irrigation which may form part of the present invention, in which a pistol-type housing 86 is provided. Housing 86 encloses device 10a and includes valve members 14 and 18, where valve member 14 includes rotatable connection port 16. Extending from housing 86 is single lumen cannula 24 which includes an outer sleeve member 26 longitudinally slidable in the direction of arrow A in the manner described above. Figure 10 illustrates a rear view of the device of Figure 8 which shows the optical fiber connection port 34a as well as connection port 20a and bayonet connector 30a.

Figure 9 illustrates surgical instrument 10a enclosed in housing 86. Connection port 20a extends in a rearward direction from body portion 12a and exits the device as shown. Coupling member 22a is provided as shown which engages body portion 12a and single lumen cannula 24, and further includes means to connect optical fiber port 34a in direct axial communication with single lumen cannula 24. Outer sleeve member 26 is slidable in a longitudinal direction in the direction of arrow A through the provision of grip member 28.

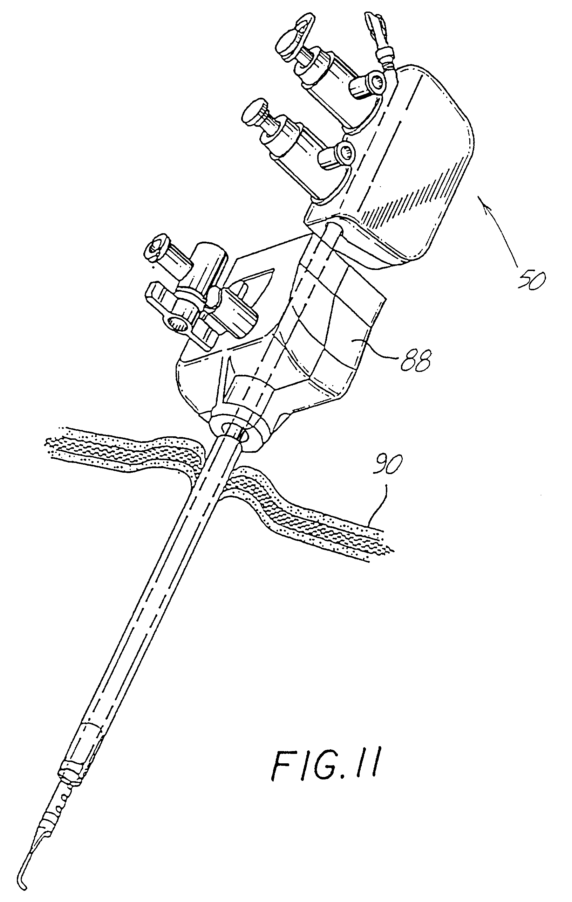

Figure 11 illustrates the suction and irrigation device 50 according to the present invention in use at a surgical site. The body wall 90 of the patient is penetrated by a trocar assembly, the cannula 88 of which remains in place after the pointed obturator has been removed. Instrument 50 is inserted to the surgical site through cannula 88 as shown to perform the surgical procedure.

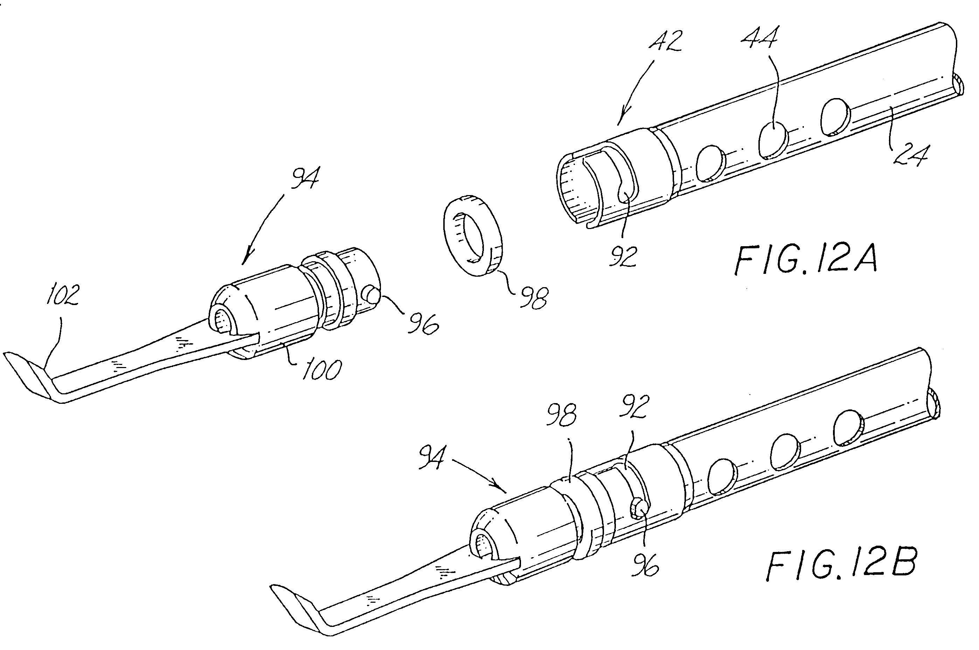

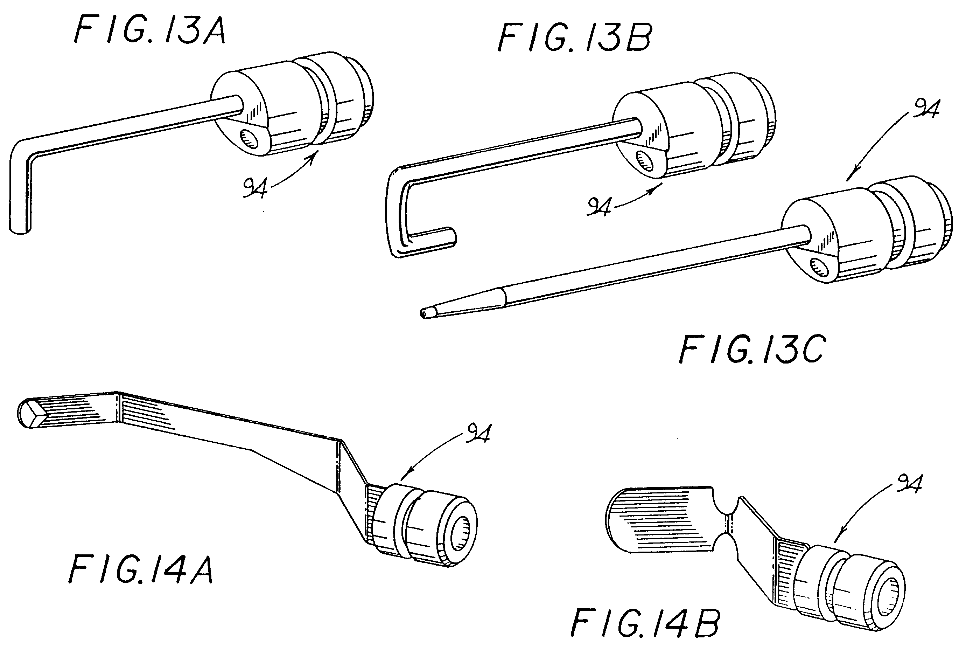

Figures 12a and 12b illustrate a novel means for connecting the working tip of the device of the present invention to the single lumen cannula. The tips may be interchangeable and may include a plurality of tips such as that shown in Figures 12a and 12b, as well as Figures 13a through 13c and Figures 14a through 14c. As shown in Figures 12a and 12b, key slots 92 are provided at the distal end 42 of single lumen cannula 24 for accepting the key posts 96 of dissector tip 94. Tip 94 is secured through a sealing gasket 98 to key slots 92. Dissector tip 94 includes a central passageway 100 to maintain fluid communication with the interior of single lumen cannula 24 adjacent the working tip 102. Tip 102 may further include a knife 104 as shown in Figure 14c.

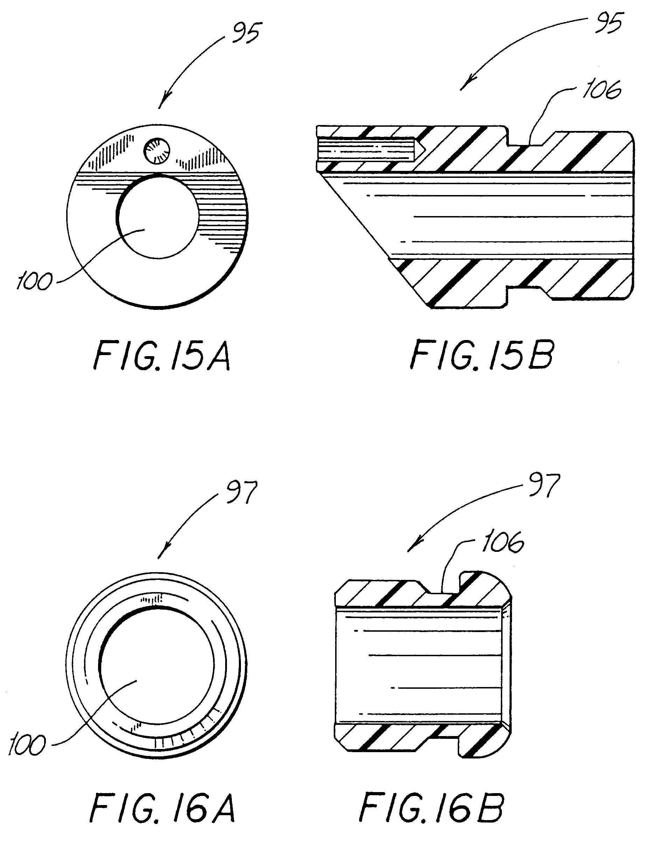

An alternate means of securing detachable dissector tip 94 to distal end 42 of single lumen cannula 24 may be accomplished through the provision of coupling members 95 and 97 as shown in Figures 15 and 16. In this embodiment, an annular groove 106 is provided which engages a detent on the interior of distal end 42 to snap fit coupling members 95 and 97 therein. In addition, a threaded connection may be used.

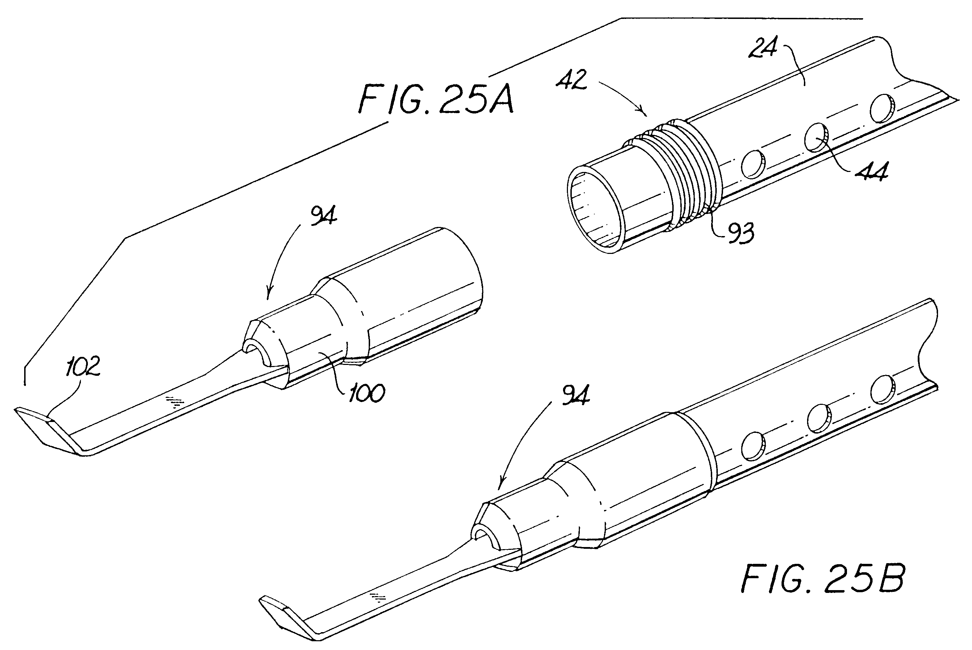

In addition, detachable tips 94 may be quickly and releasably secured to distal end 42 by means shown in Figures 25a-b and 26a-b. In Figures 25a-b, distal end 42 includes external screw threads 93, which mate with internal threads on tip 94 (not shown). Alternately, as shown in Figures 26a-b, tip member 94 may include a spring clip 110 having a deflectable post 112. Post 112 snap fits into recess 114 in distal end 42.

A further feature of the present invention provides a plurality of interchangeable tip members for connection at the distal end 42 of the suction and irrigation device 10. The interchangeable tips allows the surgeon to choose the proper working tool for the particular surgical procedure in which he is involved.

Figure 21 illustrates an embodiment of the dispenser of the present invention. Dispenser 250 comprises a top cover 252 which overlays a bottom housing 254 in a floating-type arrangement. Bottom housing 254 is provided with a plurality of access openings 256 which provide access to tool tips positioned therein. A channel 258 is provided between top cover 252 and bottom housing 254 to allow for the "floating" feature, which will be described below.

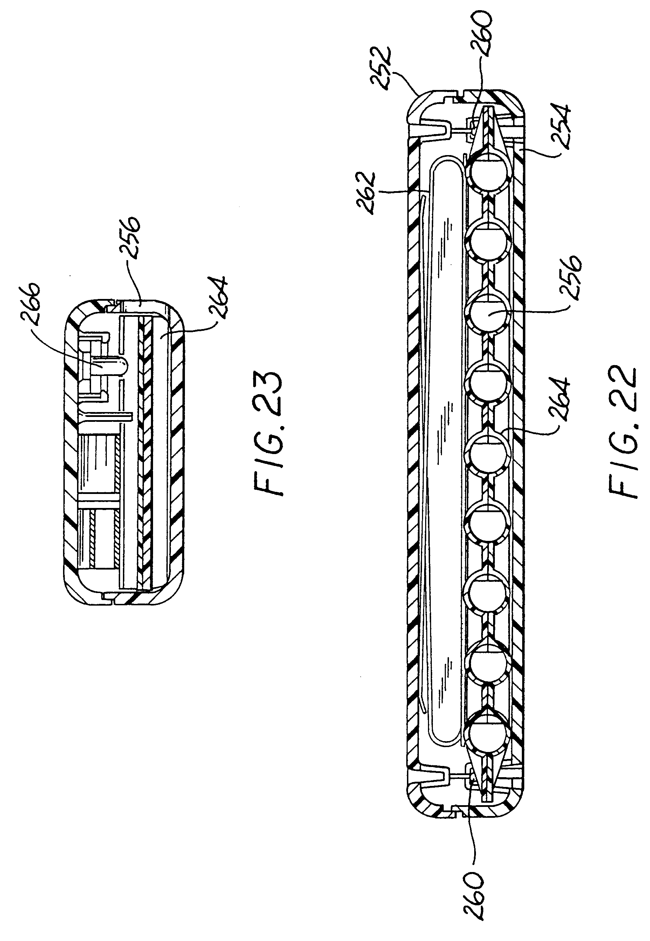

Figure 22 illustrates a cross-sectional view of the dispenser 250 taken along lines 22-22 of Figure 21. As shown, top cover 252 is secured to bottom housing 254 and floating connection point 260, which allows top cover 252 to be pushed towards bottom housing 254 to provide for manipulation of a tool member during use. Access openings 256 are formed by an internal tool frame 264 which is secured to bottom housing 254. A leaf spring member 262 is provided which facilitates the floating connection.

Figure 23 illustrates a side cross-sectional view den along lines 23-23 of Figure 21. As shown, internal tool frame 264 is aligned with access opening 256, and each of the openings 256 in internal tool frame 264 includes the provision of a release pin 266. The function of this device will now be described.

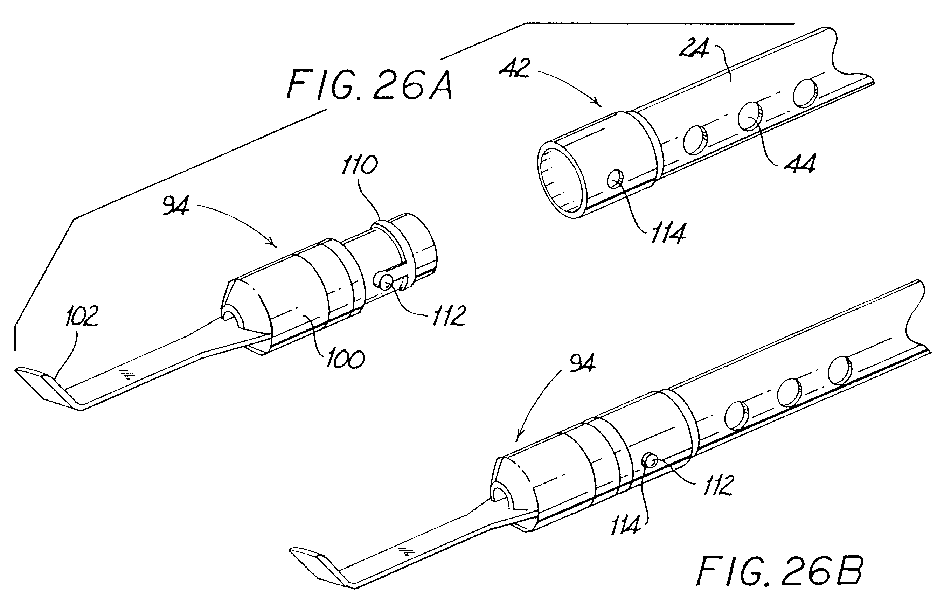

Top cover 252 is spring biased away from bottom housing 254 by leaf spring 262. The top cover includes release pin 266 which will engage spring clip 112 positioned on tool member 94 as best seen in Figures 26a and 26b. Spring clip 112 is aligned with release pin 266 but is not engaged by release pin 266 when the tip is in access opening 256. In order to remove a tip from one of the access opening 256, the surgical instrument is inserted into the dispenser so that the spring of the selected tip engages the recess 114 as shown in Figure 26b. Since the top cover 252 is biased away from the bottom housing 254, release pin 266 does not interfere with the spring. However, when it is desired to return the tip to the dispenser, top cover 252 is manually pushed towards bottom housing 254 so that release pin 266 engages spring clip 112 and pushes it out of recess 114 to disengage the instrument.

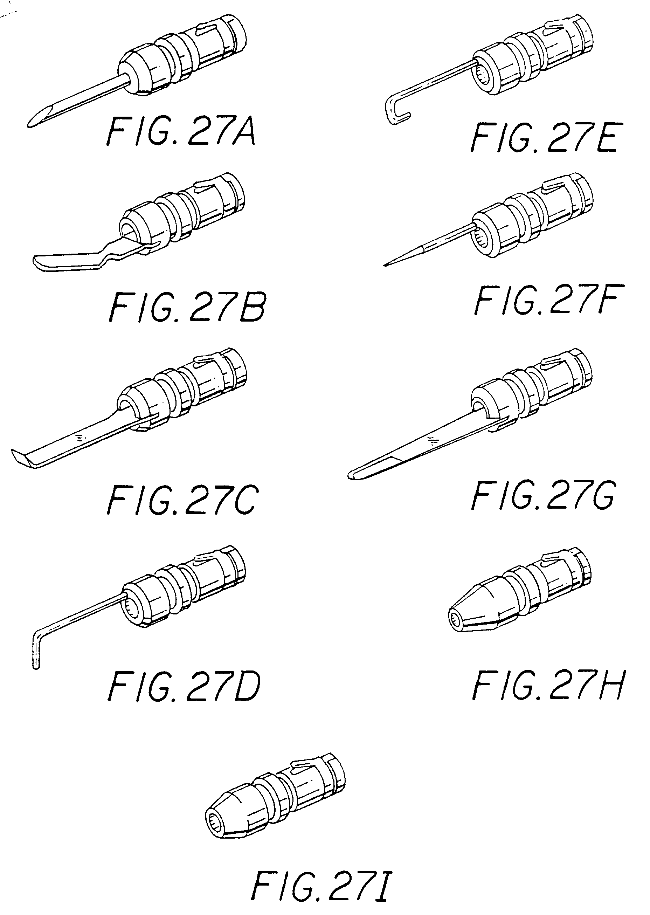

Figures 27a-27i illustrate examples of several of the tip members which may be housed in the dispenser of the present invention, such as J-hook, aqua dissector, aspiration tip, micro cautery tip, straight scalpel, L-hook, laser guide, spatula and 60° knife. Each tip includes means for coupling the tool tip to a surgical instrument; in these figures spring clip 112 is shown. The tips include electrocautery capabilities, and in particular Figures 27a, 27d, 27e and 27f each show cautery tips. Figure 27b illustrates a spatula, Figure 27c illustrates a blunt dissector, while Figure 27g shows a knife. Figures 27h and 27i each show hydrodissection nozzles.

In use, the instruments mentioned above provide a variably orientable aspiration and irrigation device which may also be used for dissecting tissue. The instrument is constructed to accommodate use with either hand and at any orientation to the patient's body during the surgical procedure through the provision of rotatable valve members which are at least 180° rotatable so that the connection hoses to the irrigation and aspiration sources may be oriented on either side of the device. Furthermore, the connection ports for at least an optical fiber for laser surgery purposes as well as a bayonet connection member for electro-cautery procedures are provided substantially parallel to the longitudinal axis of the single lumen cannula which communicates the instrument with the surgical site. In addition, one of the rotatable valve members may be replaced by a locking valve member and the connection port thereto extends directly from the distal end of the device in parallel with the longitudinal axis of the single lumen cannula.

## Claims

1. An endoscopic surgical device comprising:
an endoscopic surgical instrument (10, 10a, 50) having a handle mechanism at a first end and a coupling mechanism (22, 62; 92, 96; 95, 97) at a second end,
a surgical accessory dispenser (250) including a housing (252, 254) and a plurality of chambers disposed within said housing (253, 254), said chambers each accommodating at least one surgical accessory couplable to said coupling mechanism of said surgical instrument (10, 10a, 50), said dispenser including means (266) for uncoupling said surgical accessory from the surgical instrument (10, 10a, 50) when said accessory is within said chamber.

2. A device wherein said uncoupling means comprises a spring biased post member (266) which engages said surgical instrument (10, 10a, 50) to release said accessory.

3. A device according to any one of the preceding claims, wherein said housing includes a top portion (252) and a bottom portion (254), said top portion (252) being spring (262) biased away from said bottom portion (254), and wherein said accessory is removable from said chamber when said top portion (252) is biased away from said bottom portion (252).

4. A device according to claim 3, further comprising a chamber element (264) including a plurality of chambers, said chamber element (264) being positioned within said housing between said top (252) and bottom (254) portions.

5. A device according to claim 3 or 4, wherein said top portion (252) is movable toward said bottom portion (254) against said spring (262) bias, said top portion (252) including said means (266) for uncoupling said accessory positioned in said chamber from the surgical instrument (10, 10a, 50)

6. A device according to any one of the preceding claims, wherein said surgical accessory includes a tool member (94) for an endoscopic surgical instrument, said tool member (94) having a body portion and a coupling portion for retaining said tool member (94) in said chamber prior to attachment to said instrument (10, 10a, 50), said coupling portion also securing said tool member (94) to said instrument (10, 10a, 50).

7. A device according to any one of the preceding claims wherein the surgical accessories comprise tips for various surgical procedures.

## Patentansprüche

1. Endoskopische chirurgische Einrichtung umfassend:
- ein endoskopisches chirurgisches Instrument (10, 10a, 50) mit einem Griffmechanismus an einem ersten Ende und einem Anschlußmechanismus (22, 62; 92, 96; 95, 97) an einem zweiten Ende,
- eine Ausgabeeinrichtung (250) für chirurgisches Zubehör umfassend ein Gehäuse (252, 254) und eine Mehrzahl von Kammern, die innerhalb des Gehäuses (253, 254) angeordnet sind, wobei die Kammern alle zumindest ein chirurgisches Zubehörinstrument aufnehmen, das mit dem Anschlußmechanismus des chirurgischen Instruments (10, 10a, 50) koppelbar ist, wobei die Ausgabeeinrichtung eine Einrichtung (266) aufweist, um die chirurgischen Zubehörinstrumente von dem chirurgischen Instrument (10, 10a, 50) zu entkoppeln, wenn das Zubehörinstrument innerhalb der Kammer ist.

2. Einrichtung gemäß Anspruch 1, wobei die Entkoppeleinrichtung ein federvorgespanntes Zapfenelement (266) umfaßt, das in Eingriff tritt mit dem chirurgischen Instrument (10, 10a, 50), um das Zubehörinstrument zu lösen.

3. Einrichtung gemäß einem der vorhergehenden Ansprüche, wobei das Gehäuse einen oberen Bereich (252) und einen unteren Bereich (254) umfaßt, wobei der obere Bereich (252) von dem unteren Bereich (254) weg federbelastet (262) ist, und wobei das Zubehörinstrument von der Kammer entfernbar ist, wenn der obere Bereich (252) von dem unteren Bereich (254) weg vorgespannt ist.

4. Einrichtung gemäß Anspruch 3, weiter umfassend ein Kammerelement (264), das eine Mehrzahl von Kammern aufweist, wobei das Kammerelement (264) innerhalb des Gehäuses zwischen dem oberen (252) und unteren (254) Bereich angeordnet ist.

5. Einrichtung gemäß Anspruch 3 oder 4, wobei der obere Bereich (252) in Richtung des unteren Bereichs (254) gegen die Vorspannung der Feder (262) bewegbar ist und der obere Bereich (252) die Einrichtung (266) aufweist, um das in der Kammer angeordnete Hilfsinstrument vom chirurgischen Instrument (10, 10a, 50) zu entkoppeln.

6. Einrichtung gemäß einem der vorhergehenden Ansprüche, wobei das chirurgische Hilfsinstrument ein Werkzeugelement (94) für ein endoskopisches chirurgisches Instrument umfaßt, wobei das Werkzeugelement (94) einen Körperbereich und einen Kopplungsbereich zum Halten des Werkzeugelementes (94) in der Kammer vor der Anbringung an das Instrument (10, 10a, 50) besitzt, wobei der Kopplungsbereich auch das Werkzeugelement (94) an dem Instrument (10, 10a, 50) befestigt.

7. Einrichtung gemäß einem der vorhergehenden Ansprüche, wobei die chirurgischen Hilfsinstrumente Spitzen für verschiedene chirurgische Verfahren umfassen.

## Revendications

1. Dispositif chirurgical endoscopique comportant :
un instrument chirurgical endoscopique (10, 10a, 50) comportant un mécanisme de poignée à une première extrémité et un mécanisme d'accouplement (22, 62; 92, 96; 95, 97) à une deuxième extrémité,
un distributeur d'accessoires chirurgicaux (250) incluant un boîtier (252, 254) et plusieurs chambres disposées dans ledit boîtier (253, 254), lesdites chambres logeant chacune au moins un accessoire chirurgical apte à être accouplé audit mécanisme d'accouplement dudit instrument chirurgical (10, 10a, 50), ledit distributeur incluant un moyen (266) pour désaccoupler ledit accessoire chirurgical de l'instrument chirurgical (10, 10a, 50) lorsque ledit accessoire se trouve dans ladite chambre.

2. Dispositif, où ledit moyen de désacccouplement comporte un élément de montant (266) soumis à l'action d'un ressort qui est mis en prise avec ledit instrument chirurgical (10, 10a, 50) pour libérer ledit accessoire.

3. Dispositif selon l'une des revendications précédentes, où ledit boîtier comporte une portion supérieure (252) et une portion inférieure (254), ladite portion supérieure (252) étant sollicitée par ressort (262) au loin de ladite portion inférieure (254), et où ledit accessoire peut être retiré de ladite chambre lorsque ladite portion supérieure (252) est sollicitée au loin de ladite portion inférieure (252).

4. Dispositif selon la revendication 3, comportant en outre un élément de chambre (264) incluant plusieurs chambres, ledit élément de chambre (264) étant positionné dans ledit boîtier entre lesdites portions supérieure (252) et inférieure (254).

5. Dispositif selon la revendication 3 ou 4, où ladite portion supérieure (252) est déplaçable vers ladite portion inférieure (254) contre ladite sollicitation par ressort (262), ladite portion supérieure (252) incluant ledit moyen (266) pour désaccoupler ledit accessoire positionné dans ladite chambre de l'instrument chirurgical (10, 10a, 50).

6. Dispositif selon l'une des revendications précédentes, où ledit accessoire chirurgical comporte un élément d'outil (94) pour un instrument chirurgical endoscopique, ledit élément d'outil (94) ayant une portion de corps et une portion d'accouplement pour retenir ledit élément d'outil (94) dans ladite chambre avant l'attachement audit instrument (10, 10a, 50), ladite portion d'accouplement fixant également ledit élément d'outil (94) audit instrument (10, 10a, 50).

7. Dispositif selon l'une des revendications précédentes, où les accessoires chirurgicaux comportent des pointes pour divers procédés chirurgicaux.
